# EUROPEAN PATENT SPECIFICATION

(11) **EP 0 514 934 B1**
(45) Date of publication and mention of the grant of the patent: **10.04.1996**
(21) Application number: 92108702.9
(22) Date of filing: 22.05.1992
(51) Int. Cl.: A61K 7/48, A61K 7/06, A61K 7/00

(54) **Optically clear hair care compositions containing silicone based microemulsions**
Optisch klare Haarpflegemittel enthaltend Silicon-Mikroemulsion
Composition pour le soin des cheveux optiquement claire contenant des microémulsions de silicone

(30) Priority: 24.05.1991 US 705455
(43) Date of publication of application: 25.11.1992
(73) Proprietor: DOW CORNING CORPORATION, Midland Michigan 48686-0994 (US)
(72) Inventor: Halloran, Daniel Joseph, Midland, Michigan (US)
(74) Representative: Spott, Gottfried, Dr.

(56) References cited:
- EP-A- 0 138 192
- EP-A- 0 180 464
- EP-A- 0 268 982
- EP-A- 0 270 249
- EP-A- 0 363 252
- US-A- 5 063 044

## Description

This invention relates to the use of silicone based microemulsions in hair care compositions such as conditioners and shampoos and is more particularly directed to compositions and methods for treating hair with aminofunctional microemulsions of the nonionic, cationic or anionic types that result in an optically clear hair care composition.

It is desirable to provide hair care compositions which have beneficial effects on wet and dry hair, for example, which improve handle, softness, silkiness and ease of combing the hair. Such beneficial effects may be obtained by incorporating certain types of silicones into the hair care compositions. The preferred silicones include polydiorganosiloxanes wherein the organic substituents are alkyl or aryl groups. However, such compounds are not easily incorporated and result in opaque or at best translucent hair care products. It is aesthetically desirable to provide optically clear hair care compositions.

Silicones in hair care compositions have been disclosed in a number of different publications. For example, U.S. Patent No. 4,788,006 issued November 29, 1988, teaches shampoo compositions which comprise a synthetic, anionic surfactant, a dispersed, insoluble, non-volatile silicone, a xanthan gum suspending agent and water.

Hair care compositions containing polyorganosiloxanes are well known in the hair care art and need not be further described here. Hair treating compositions containing amino functional polysiloxanes have also been described in several publications. For example, U.S. Patent No. 4,563,347 issued January 7, 1986 teaches that an aqueous emulsion of aminoalkyl substituted polydimethylsiloxane is useful to condition hair because it facilitates combing and imparts a smooth feel to hair. The aminoalkyl substituents are credited with providing the copolymers with cationic sites that make the polymer more substantive to hair than nonsubstituted polydimethylsiloxane. The '347 patent further teaches the use of aminofunctional polydiorganosiloxane solutions and emulsions as conditioners. This is in contrast to the concept of the present invention which utilizes aminofunctional microemulsions in formulations to produce clear and stable hair care compositions. Other hair treating compositions containing amino functional polysiloxanes are described in U.S. Patent Nos. 4,586 518, 4,601,902 and 4,618,689, however, these references do not describe the use of microemulsions in these compositions.

Amino functional microemulsions have also been described in the hair care art. For example, U.S. Patent No. 4,749,732 issued June 7, 1988 discloses hair care uses of polydiorganosiloxanes containing aminoalkyl groups modified by alkoxycarbonylalkyl substituents. The '732 patent also teaches that the modified aminoalkyl silicones exhibit improved deposition on hair and can be formulated into shampoos or conditioners. The '732 patent states that microemulsions of modified aminoalkyl substituted polydiorganosiloxane can be prepared by the method described in U.S. Patent No. 4,620,878 issued November 4, 1986 which describes generally the preparation of emulsions of silicones containing polar substituents. The '732 patent does not disclose how to prepare these modified aminoalkyl substituted polydiorganosiloxane microemulsions. Further the '732 patent does not disclose an unmodified or unsubstituted amino functional microemulsion as an ingredient in an optically clear hair care formulation. In addition, these modified amino substituted microemulsions utilize acrylic monomers which give rise to serious odor problems making the materials unsuitable for cosmetic use. The '878 patent teaches a method of preparing clear microemulsions of amine functional polyorganosiloxanes. It is further disclosed in Example 24 of the '878 patent that transparent microemulsions of amine functional siloxanes can be mixed with a shampoo base of sodium laurel ether sulfate and water to produce a stable, clear composition. However, the '878 patent does not teach an amino functional microemulsion as an ingredient present in an optically clear and operative hair care formulation.

Example II and Comparative Example I of this specification clearly illustrate the unexpected advantages of this invention over the composition described in Example 24 of the '878 patent.

In U.S. Patent No. 4,559,227 issued December 17, 1985, a conditioning shampoo composition is taught containing a nonionic surfactant of the alkanolamide or amine oxide type, an amine functional polydiorganosiloxane as a hair conditioning component, a detersive surfactant of the anionic or amphoteric type and water, the shampoo composition being in the form of an aqueous solution. The '227 patent further discloses a method of preparing the shampoo composition as a stable solution. This is in contrast to the concept of the present invention which employs premade microemulsions as opposed to solutions as components to provide optically clear hair care formulations. The use of the premade microemulsion allows an easier to use shampoo to be formed without a complex premixing of the composition.

Polydiorganosiloxane microemulsions have also been described in the hair care art. For example, European Patent Publication No. 0 268 982 A2 dated January 6, 1988 is directed to cosmetic compositions containing microemulsions of dimethylpolysiloxanes produced by emulsion polymerization which provide long term storage stability. However, the formulations of the '982 application do not disclose an amino functional silicone microemulsion as an ingredient in an optically clear hair care formulation that provides substantive conditioning. In United States application for patent, Serial No. 481,003, filed February 16, 1990 and assigned to the same assignee as this present application, which teaches a hair conditioning composition being a mixture including water, a thickener and an organosilicon compound. The organosilicone compound can take the form of a microemulsion and is selected from the group consisting of carboxy-glycol ether and carboxy-glycol ester functional polysiloxanes, which are appreciably polar and soluble molecules. In contrast, the present invention utilizes a non-polar insoluble aminofunctional polydiorganosiloxane microemulsions in combination with a deposition aid to unexpectedly provide true optical clarity to the hair care compositions of this invention along with robust, substantive conditioning.

The present invention can therefore be distinguished from the references enumerated above in that the present invention contains organofunctional silicone based microemulsions as ingredients in combination with a deposition aid to provide truly optically clear hair care compositions that have beneficial effects greater than the hair care formulations known in the art and in view of the fact that the cationic and nonionic lipophilic compounds of the prior art do not generally deposit well from hair care compositions containing anionic detersive surfactants, it is surprising that the amine functional microemulsion and the cationic or nonionic lipophilic compounds (as part of the microemulsion or separately) are so effectively deposited from the hair care compositions of the present invention which can contain anionic detersive surfactants and at the same time retains clarity and stability. Therefore, since the hair care compositions of the present invention provide a unique and advantageous combination of superior foaming and substantive conditioning in one clear and easy to use product they are advantageous over the formulations of the art.

This invention is directed to optically clear hair care compositions containing amino-functional silicone based microemulsions which are of the anionic, cationic and nonionic types, a deposition aid, a cleaning agent, water and a water-thickening polymer and/or electrolyte. Beneficial advantages obtained from the hair care formulations of the present invention include clarity, ease of use, outstanding conditioning, easy formulatability, excellent foaming and detergent compatability. While not limiting the present invention with any particular theory, it is believed that the microemulsion must contain a siloxane with polar functionality to allow a truly clear hair care composition to be formed. A particularly effective polar functionality is an amine, since the amine functionality functions both to give clear systems and to give substantive conditioning. The clarity is believed to be achieved through improved interfacial tension reduction due to the presence of the amine groups. Microemulsions generally do not deposit well from anionic detergent formulations, due to their excellent stability. It has been found that cationic or nonionic lipophilic compounds act as deposition aids for the microemulsions. The deposition aid co-deposits with the organofunctional siloxane microemulsion. The deposition of the microemulsion is enhanced due to the use of the deposition aid. Surprisingly, the presence of the deposition aid does not interfere with the clarity, stability and/or the substantive properties of the silicone microemulsion. It is believed that the deposition aid enhances the deposition of the microemulsion by codepositing with it and the codeposition is enhanced by the electrostatic or lipophilic attractions of the deposition aid for the hair.

These and other features, objects and advantages of the herein described present invention will become more apparent when considered in light of the following detailed description.
The present invention relates to
an optically clear hair care composition comprising:
(A) a polyorganosiloxane microemulsion;
(B) a deposition aid selected from the group consisting of non-polar oils selected from mineral oils, alkanes, or naturally derived oils, silicones selected from the group consisting of cyclomethicone, dimethicone, amodimethicone polymers, and dimethicone copolyols, resins, salts selected from the group consisting of a nonpolar quaternary ammonium salt having its formula selected from the group consisting of 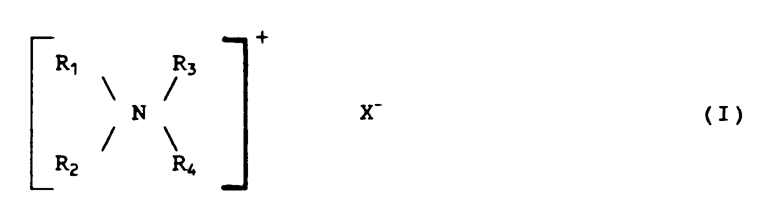 wherein R₁ is selected from the group consisting of an aliphatic group of from 12 to 22 carbon atoms, an aromatic group having from 12 to 22 carbon atoms, an aryl group having from 12 to 22 carbon atoms, and an alkylaryl group having from 12 to 22 carbon atoms, R₂ is an aliphatic group having from 12 to 22 carbon atoms, R₃ and R₄ are each alkyl groups having from 1 to 3 carbon atoms, and X is an anion selected from halogen, acetate, phosphate, nitrate and alkylsulfate radicals, and 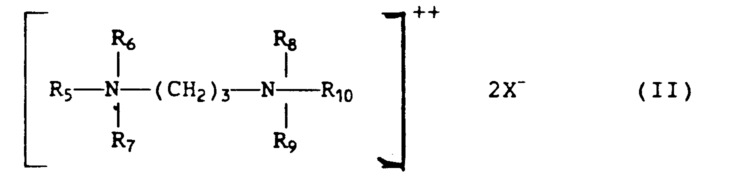 wherein R₅ is an aliphatic group having from 16 to 22 carbon atoms, R₆, R₇, R₈, R₉, and R₁₀ are selected from the group consisting of hydrogen and alkyl radicals having from 1 to 4 carbon atoms, and X is an ion selected from the group consisting of halogen, acetate, phosphate, nitrate, and alkyl sulfate radicals, amine salts, sodium chloride, ammonium chloride, sodium sulfate, and low molecular weight organic salts; and
(C) water;
   wherein the polyorganosiloxane microemulsion comprises a radical selected from the group consisting of a polar radical attached to Si through Si-C or Si-O-C bonds and a silanol radical; wherein the microemulsion additionally comprises a surfactant which is insoluble in the polyorganosiloxane and water; wherein the polyorganosiloxane in the microemulsion has an average particle size of less than 0.14 µm (microns)
For the purposes of the present invention, the term "optically clear" is used to define a composition that is transparent (transmitting light without distortion). Which means that the size of the particles in the composition are reduced to a size where they are not observable with optical (visual) means. According to the present invention, "optically clear" is further defined by NTU's (Nephelometric Turbidity Units), which is the unit of measure for the turbidity or haze of a liquid. NTU's range from 0.04 to 1,000 or higher. A more detailed description of this test is found hereinbelow. The haze value of a relatively turbid solution is about 100 NTU's or higher and mixtures with a slight haze give values of 20 to 50 NTU's. In contrast, the compositions of the present invention have an average haze value of 3 to 5 NTU's.

Microemulsions are mixtures of oil and water where the particle size of the resulting droplets is small enough so the resulting mixture is clear or translucent. Because of their relative clarity microemulsions are distinguishable from standard opaque emulsions in that certain microemulsions can be used to prepare clear cosmetics. The clarity of these compositions is advantageous in cosmetic applications such as in the hair care art. Microemulsions are also more temperature, dilution and formulation stable than standard emulsions. Microemulsions droplet sizes are variously defined in the chemical art with an upper limit on the droplet size typically being placed somewhere between 0.10 and 0.15 micron to distinguish microemulsions from opaque standard emulsions. In general, microemulsions can also be defined by their appearance: microemulsions are transparent or translucent and do not display the opalescence of standard emulsions. While microemulsions with average droplet sizes between 0.10 and 0.15 micron display the properties of microemulsions, microemulsions with average droplet sizes less than 0.10 micron are preferred for their even greater clarity and stability.

The cosmetics and toiletry industry has produced a wide range of grooming aids for use by men and women. Among the myriad of such products are shampoos to clean the hair and scalp and hair conditioners to facilitate the combing out of the hair and impart softness and suppleness to the hair. Cleanliness, of the hair and scalp are important personal grooming criteria. Extensively soiled hair takes on a lackluster appearance and becomes oily and gritty to the touch. The shampoo must foam quickly and copiously and rinse out thoroughly leaving the hair with a fresh clean scent, soft, shiny, lustrous, full bodied and in a manageable state. While most shampoos are clear liquids or opaque lotions, they are available as clear gels, cream pastes and aerosols. Shampoos contain one or more cleaning agents such as anionic, nonionic, amphoteric or ampholytic and cationic surfactants; and various additives including viscosity control agents, opacifiers, conditioners, preservatives and fragrances. Wet hair is often tangled and difficult to comb following shampooing and for this reason it is common to apply rinses and conditioners in order to enhance ease of combing, detangling, body, shine, texture, prevention of static buildup and manageability. Creme rinses and combination creme rinse conditioners also generally improve the finish of hair.

In accordance with the present invention, a new hair care formulation of any of the above mentioned categories of hair care products is provided and which includes an amino functional silicone microemulsion.

As noted hereinabove, the hair care formulations of this invention include three or optionally five ingredients. The essential ingredients are a polyorganosiloxane microemulsion, a deposition aid (optionally as part of the microemulsion) and water. Optionally, the composition can additionally comprise a cleaning agent of the anionic or nonionic surfactant types and/or a water-thickening agent selected from electolytes, polyelectrolytes and/or water soluble polymers.

Component (A) in the compositions of this invention comprises a polyorganosiloxane microemulsion. The microemulsions of this invention comprise: (1) a polyorganosiloxane which contains at least one polar radical attached to Si through Si-C or Si-O-C bonds or at least one silanol radical, (2) a surfactant which is insoluble in the polyorganosiloxane and (3) water wherein the polyorganosiloxane in the microemulsion has an average particle size of less than 0.14 microns and wherein the polyorganosiloxane microemulsion is transparent or translucent. The polyorganosiloxanes useful in this invention must contain at least one polar radical attached to silicon through a silicon-carbon bond or a silicon-oxygen carbon bond or at least one silanol radical. The polyorganosiloxanes should be liquid at the temperature at which the oil concentrate is prepared. Suitable polar radicals may contain substituents such as amines, amine salts, amides, carbinols, carboxylic acids, carboxylic acid salts, phenols, sulfonate salts, sulfate salts, phosphate acids and phosphate acid salts, where the polar radical is attached to silicon through silicon-carbon or silicon-oxygen-carbon bonds or the polar radicals may be hydroxyl radicals. Naturally if the polar radical is a hydroxyl radical then the polyorganosiloxane contains silanol radicals or groups. The siloxane unit which contains the polar radical may be illustrated by the general formula RₐQ_{b}SiO_{(4-a-b)/2} where a is 0 to 2, b is 1 to 3 with the sum of (a+b) being less than or equal to 3, R is a monovalent hydrocarbon or substituted hydrocarbon radical and Q is a polar radical. The polyorganosiloxane may contain additional siloxane units of general formula R_{c}SiO_{(4-c)/2} where c is 1 to 3 and R is a monovalent hydrocarbon or substituted hydrocarbon radical. Illustrative of the R radicals that can be present are alkyl radicals such as the methyl, ethyl, propyl, butyl, amyl, hexyl, octyl, decyl, dodecyl, octadecyl and myricyl radicals; allenyl radicals such as the vinyl, allyl and hexenyl radicals; cycloalkyl radicals such as the cyclobutyl and cyclohexyl radicals; aryl radicals such as the phenyl, xenyl and naphthyl radicals; aralkyl radicals such as the benzyl and 2-phenylethyl radicals; alkaryl radicals such as the tolyl, xylyl and mesityl radicals; the corresponding halohydrocarbon radicals such as 3-chloropropyl, 4-bromobutyl, 3,3,3-trifluoropropyl, chlorocyclohexyl, bromophenyl, chlorophenyl, a,a,a-trifluorotolyl and the dichloroxenyl radicals; the corresponding cyanohydrocarbon radicals such as 2-cyanoethyl, 3-cyanopropyl and cyanophenyl radicals; and the corresponding mercaptohydrocarbon radicals such as mercaptoethyl, mercaptopropyl, mercaptohexyl and mercaptophenyl. It is preferred that R be a hydrocarbon radical containing from 1 to 18 carbon atoms. Especially preferred R radicals are methyl, phenyl and vinyl radicals.

The preferred polyorganosiloxanes of this invention may be illustrated by the general formula

R_{(3-d)}Q_{d}Si(R₂SiO)ₓ(RQSiO)_{y}SiR_{(3-d')}Q'_{d'}

where R is a monovalent hydrocarbon or substituted hydrocarbon radical as defined above, Q is a polar radical attached to Si through Si-C or Si-O-C bonds or the -OH radical and d and d' are, independently 0, 1, 2, 3. The values of x and y are not particularly limited so long as ($\text{y+d+d'}$) is at least one and the sum (x + y) is not so large that the oil concentrate cannot be dispersed rapidly enough to obtain a microemulsion with an average particle size of less than 0.14 microns. It is also preferred that both d and d' are zero so that the polyorganosiloxanes are endblocked with triorganosiloxy units. Again, especially preferred R radicals are methyl, phenyl and vinyl radicals.

Other polyorganosiloxanes may be employed in the practice of this invention so long as they have at least one polar radical attached to silicon through silicon-carbon or silicon-oxygen-carbon bonds or at least one silanol radical. As indicated earlier, suitable polar radicals may contain amine, amine salt, amide, carbinol, carboxylic acid, carboxylic acid salt, phenol, sulfonate salt, sulfate salt, phosphate acid and phosphate salt substituents. The polar radical may also be a hydroxyl radical. Except for the hydroxyl radical, all of the polar radicals should be attached to silicon through a silicon-carbon bond or through silicon-oxygen-carbon bonds. It is preferred that the polar radical be attached to silicon through the Si-C bond. Generally these polar radicals, except for hydroxyl, should have the general formula -R'G where R' is a divalent linking group composed of carbon and hydrogen atoms; carbon, hydrogen and oxygen atoms; or carbon, hydrogen and sulfur atoms; and where G is a polar radical. Specific examples of R' include the methylene, ethylene, propylene, hexamethylene, decamethylene, -CH₂CH(CH₃)CH₂-, phenylene, napthylene, -CH₂CH₂SCH₂CH₂-, -CH₂CH₂OCH₂-, -OCH₂CH₂-, -OCH₂CH₂CH₂-, 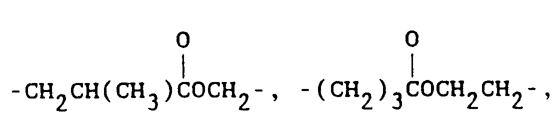
C₆H₄C₆H₄-, C₆H₄CH₂C₆H₄-, and the 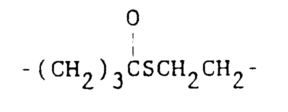
radical. It is preferred that the R' linking group contain from 2 to 10 carbon atoms. It is post preferred that the R' linking group contains from 3 to 4 carbon atoms. When the polyorganosiloxane is an amine functional siloxane the polar radical is preferably illustrated by the general formula -R'NHR² wherein R' is the divalent linking group discussed above and R² is selected from the group consisting of hydrogen atom, alkyl radicals containing from 1 to 4 carbon atoms and -CH₂CH₂NH₂ radical. The most preferred amine-functional polar radicals are -CH₂CH₂CH₂NHCH₂CH₂NH₂ and -CH₂CH(CH₃)CH₂NHCH₂CH₂NH₂. Salts of these same aminefunctional radicals may also be used in the present invention. Examples of such salts include alkyl carboxylate salts, aryl carboxylate salts, halide salts such as chlorides and bromides and other neutralization products of the amines with organic acids.

Two types of amide-functional polar radicals may be illustrated by the general formulae 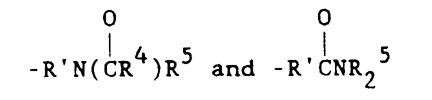
where R' is the divalent linking group discussed above, R⁴ is a monovalent alkyl radical containing from 1 to 6 carbon atoms and R⁵ is selected from the group consisting of hydrogen, alkyl radicals containing from 1 to 4 carbon atoms and 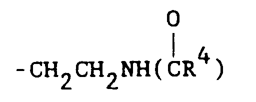
radicals. Preferably, the amide functional radicals used in the present invention are

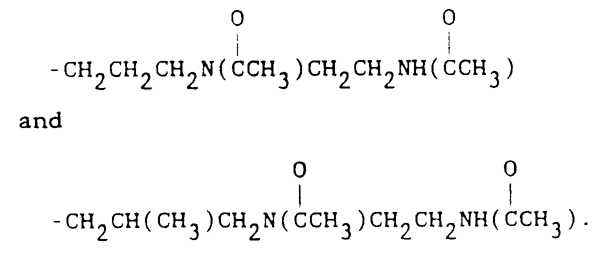

The polar radical on the polyorganosiloxane may also be in the form of a carbinol. In general, examples of suitable carbinol radicals may be represented by the general formula -R'O(C₂H₄O)ₑ(C₃H₆O)_{f}H where R' is the divalent linking group discussed above and e and f are both greater than or equal to zero. When both e and f equal zero, the carbinol radicals are the simple alcohol radicals -R'OH. When e is greater than zero the carbinol contains an ethylene glycol portion; where f is greater than zero the carbinol contains a propylene glycol portion. Other suitable polar radicals or substituents include carboxylic acids and their salts. These polar radicals may contain one or more COOH groups or their salts and can generally be expressed by the formula -R'COOH where R' is the divalent linking group. Examples of the cations capable of forming carboxylic acid salts suitable for use in this invention include Na+, K+, Li+, NH₄+ and pyridinium ions. Preferred carboxylic acid polar radicals include -CH₂CH₂SCH₂COOH, -(CH₂)₁₀COOH and -CH₂CH₂SCH₂COOX where X is selected from the group consisting of Na+, K+, Li+ and NH₄+.

The polar radicals may also be of the phenol type expressed by the general formula R'C₆H_{w}(OH)_{5-w} where R' is the divalent linking group and where w is 0 to 4. Polyaromatic rings substituted with hydroxyl radicals are also suitable polar substituents for the practice of this invention.

Other polar radicals or substituents suitable for incorporation in the polyorganosiloxanes of this invention include the sulfonic acid salts and sulfate salts. Examples of such polar radicals are illustrated by the general formula -R'SO₃X for the sulfonic acid salt and -R'OSO₃X for the sulfate salt where X is a suitable cation such as Na+, K+, Li+ and NH₄+.

The polar radicals may also be in the form of phosphate acids and phosphate acid salts of the general formula -R'P(OH)₂O or -R'P(OX)₂O, respectively, where X is a suitable cation such as Na+, K+, Li+ and NH₄+.

Polyorganosiloxanes which contain silanol radicals, SiOH, may also be emulsified by the method of this invention.

Examples of such silanol containing polyorganosiloxanes include linear hydroxyl end-blocked polyorganosiloxane, branched hydroxyl end-blocked copolymers of diorganosiloxane and monoorganosiloxane, as well as polyorganosiloxanes where the hydroxyl radicals are found along the siloxane chain.

The specific recitation of the various polar radicals suitable for incorporation in the polyorganosiloxanes of this invention is not intended as a limitation of this invention. As one skilled in the art would realize polar radicals containing amines, amine salts, amides, carbinols, carboxylic acids, carboxylic acid salts, phenols, sulfonate salts, sulfate salts, phosphate acids and phosphate acid salts, as well as hydroxyl radicals other than those specifically described herein would be suitable polar radicals for the polyorganosiloxanes of this invention. It will also be realized by one skilled in the art that the polyorganosiloxanes suitable for use in this invention may have more than one type of polar radical in the molecule. One skilled in the art should also realize that polyorganosiloxanes which have more than one type of polar radical in a single substituent would also be suitable for use in this invention.

As indicated earlier, the polyorganosiloxane must have at least one polar radical. It is preferred that the polyorganosiloxanes have from about 0.1 to 15 molar percent of the described polar radicals and most preferably from about 0.2 to 10 molar percent of the described polar radical. The polar radical containing polyorganosiloxanes useful in the present invention can be prepared by procedures well known in the art. Many of these polyorganosiloxanes are available commercially. Therefore, their preparation will not be described here. Two methods were utilized in preparing the microemulsions that are employed in the hair care compositions according to this invention. The first is a mechanical method described in U.S. Patent No. 4,620,878 issued November 6, 1986. The method involves forming a "translucent concentrate" of surfactant, polysiloxane and water in select proportions. The "concentrate" is then rapidly dispersed in additional water to form the microemulsion. The second method by which the microemulsions of the present invention are produced is described in United States application for patent Serial. No. 532,471, filing date June 1, 1990 and assigned to the same assignee as this present application, which teaches a method of producing oil free microemulsions by emulsion polymerization, the method comprising a mixture of at least one siloxane oligomer, cationic or anionic surfactant, nonionic surfactant, catalyst and water whereby the siloxane oligomer is reacted in the presence of water and the surfactants to form the polysiloxane microemulsions. It is further taught that although the order the ingredients are combined in is not critical, it is essential to have agitation during and following the addition of the ingredients and to have achieved or to heat to the polymerization temperature when all the ingredients have been combined. The amine functional microemulsions of this invention should be present in the hair care compositions in an amount from about 3 to 30 weight percent or more preferably about 4 to 9 weight percent based on the total weight of the composition. At concentrations below the minimum concentration disclosed herein, the amount of microemulsion that is deposited on the hair is insufficient to impart any significant conditioning to the hair. Concentrations greater than the maximum concentration disclosed are inefficient because an overabundance of emulsion deposited on the hair results in the hair having a greasy feel due to it being in an over conditioned state. For the hair care compositions to be optically clear, the Cationic Equivalence of the microemulsions are preferably less than 0.14. For purposes of the present invention, the term "Cationic Equivalence" is used to define the number of equivalents or milliequivalents (depending on the data source) of cationic functionality per gram of sample. It is critical to the compositions of this invention that a microemulsion be present to provide clear hair care compositions. Component (B) in the compositions of this invention is a deposition aid. In accordance with the present invention, the deposition aid is any material which improves the deposition onto the hair of a silicone-based microemulsion.

The deposition aids can be nonpolar oils such as mineral oils and/or other oils such as alkanes, vegetable oils such as sweet almond oil, avocado oil, apricot kernel oil, sesame oil and olive oil or other naturally derived oils or isoparrafins such as isooctane or silicones such as cyclomethicone and dimethicone or silicone organic copolymers such as amodimethicone polymers and dimethicone copolyol. These oils can be applied in neat or in emulsified form. The deposition aid may consist of resins.

The deposition aid can be cationic and is preferred to be cationic in certain embodiments. Cationic materials useful in the compositions of the present invention as deposition aids contain amino or quaternary ammonium hydrophilic moieties which are positively charged when dissolved in the aqueous composition of the present invention. Cationic vehicle materials among those useful herein are disclosed in the following documents: M.C. Publishing Co., McCutcheon's, Detergents and Emulsifiers, p. 257-258, (North American edition 1989); Schwartz et al., Surface Active Agents, Their Chemistry and Technology, New York: Interscience Publishers, p. 151-210 (1949); and U.S. Patent No. 4,387,090, Bolich Jr., issued June 7, 1983.

Among the quaternary ammonuim-containing cationic materials useful herein are those of the general formula: 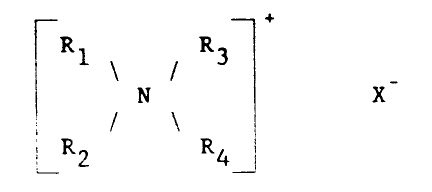
wherein R₁ is hydrogen, an aliphatic group of from 1 to 22 carbon atoms or an aromatic, aryl or alkylaryl group having from 12 to 22 carbon atoms; R₂ is an aliphatic group having from 1 to 22 carbon atoms; R₃ and R₄ are each alkyl groups having from 1 to 3 carbon atoms and X is an anion selected from halogen, acetate, phosphate, nitrate and alkylsulfate radicals. The aliphatic groups may contain, in addition to carbon and hydrogen atoms, ether linkages and other groups such as amido groups.

Other quaternary ammonium salts useful herein are of the formula: 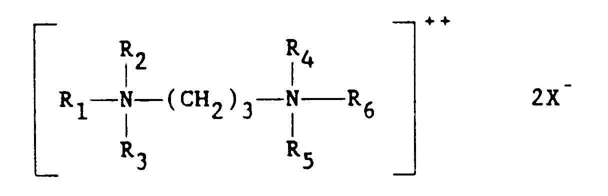
wherein R₁ is an aliphatic group having from 16 to 22 carbon atoms, R2, R3, R4, R5 and R6 are selected from hydrogen and alkyl having from 1 to 4 carbon atoms and X is an ion selected from halogen, acetate, phosphate, nitrate and alkyl sulfate radicals. Such quaternary ammonium salts include tallow propane diammonium dichloride.

Preferred quaternary ammonium salts include dialkyldimethylammonium chlorides, wherein in the alkyl groups have from 12 to 22 carbon atoms and are derived from long chain fatty acids, such as hydrogenated tallow fatty acid. (Tallow fatty acids give rise to quaternary compounds wherein R₁ and R₂ have predominantly from 16 to 18 carbon atoms.) Examples of quaternary ammonium salts useful in the present invention include ditallowdimethyl ammonium chloride, ditallowdimethyl ammonium methyl sulfate, dihexadecyl dimethyl ammonium chloride, di(hydrogenated tallow) dimethyl ammonium chloride, dioctadecyl dimethyl ammonium chloride, dieicosyl dimethyl ammonium chloride, didocosyl dimethyl ammonium chloride, di(hydrogenated tallow) dimethyl ammonium acetate, dihexadecyl dimethyl ammonium chloride, dihexadecyl dimethyl ammonium acetate, ditallow dipropyl ammonium phosphate, ditallow dimethyl ammonium nitrate, di(coconutalkyl) dimethyl ammonium chloride and stearyl dimethyl benzyl ammonium chloride. Ditallow dimethyl ammonium chloride, dicetyl dimethyl ammonium chloride, stearyl dimethyl benzyl ammonium chloride and cetyl trimethyl ammonium chloride are preferred quarternary ammonium salts useful herein. Di(hydrogenated tallow) dimethyl ammonium chloride is a particularly preferred quarternary ammonium salt. Salts of primary, secondary and tertiary fatty amines are also preferred cationic vehicle materials. The alkyl groups of such amines preferably have from 12 to 22 carbon atoms and may be substituted or unsubstituted. Secondary and tertiary amines are preferred, tertiary amines are particularly preferred. Such amines, useful herein, include stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E. O. ) stearylamine, dihydroxy ethyl stearylamine and arachidylbehenylamine. Suitable amine salts include the halogen, acetate, phosphate, nitrate, citrate, lactate and alkyl sulfate salts. Such salts include stearylamine hydrochloride, soyamine chloride, stearylamine formate and N-tallowpropane diamine dichloride and stearamidopropyl dimethylamine citrate. Cationic amine compounds included among those useful in the present invention are disclosed in U.S. Patent No. 4,275,055, Nachtigal et al., issued June 23, 1981.

Amphoteric compounds useful as deposition aids in the present invention include, among others, cocoamphocarboxyglycinate, cocoamphocarboxypropionate cocobetaine, N-cocamidopropyldimethylglycine and N-lauryl-N'- carboxymethyl-N' -(2-hydroxyethyl)ethylenediamine. Other suitable amphoteric compounds include the quaternary cycloimidates, betaines, sultaines disclosed in U.S. Patent No. 3,964,500. The quaternary cycloimidates have the general structure: 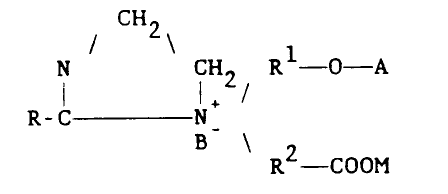
wherein R is an aliphatic hydrocarbon radical having about 9 to about 17 carbon atoms; R¹ and R² are each dependently (a) a divalent alkylene radical having 1 to 4 carbon atoms, (b) a hydroxy-substituted divalent alkylene radical having 2 to 4 carbon atoms, (c) a divalent alkylene radical having 2 to 4 carbon atoms wherein said alkylene radical contains an ether or a keto linkage and (d) a hydroxy-substituted divalent alkylene radical having 2 to 4 carbon atoms wherein said alkylene radical contains an ether or a keto linkage; M is a water-solubilizing cation; A is (a) M, (b) -CH₂COOM, (c) -C₂H₄OCH₂COOM or (d) -C₂H₄COOM; and B is (a) OH⁻, (b) C₁₂H₂₅OSO₃⁻ or (c) C₁₂H₂₅-C₆H₄-SO₃⁻.

Particularly preferred amphoteric compounds are the substituted quaternary hydroxy cycloimidinic acid alkali metal alcoholates described in U.S. Patent No. 2,528,378 and which have the generic structure: 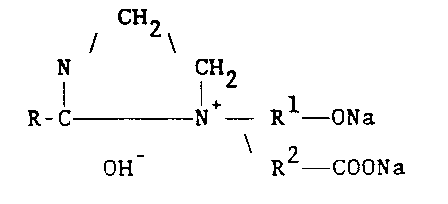
wherein R is an aliphatic hydrocarbon radical having about 9 to 17 carbon atoms, R¹ and R² represent divalent alkylene groups having 1 to 4 carbon atoms and may be the same or different.

The most preferred of the amphoteric compounds are the substituted quaternary hydroxy cycloimidinic acid alkali metal alkoxymethyl carboxylates described in U.S. Patent No. 2,781,354 and which have the generic structure: 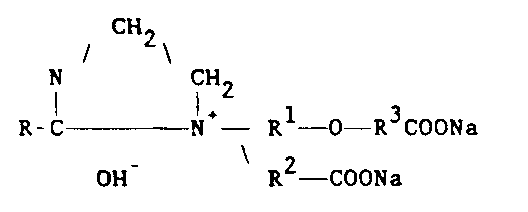
wherein R is an aliphatic hydrocarbon radical having about 9 to about 17 carbon atoms, R¹ and R² are as defined above and R³ is a divalent alkylene group having 1 to 2 carbon atoms. A preferred compound is one having the foregoing structure wherein R has 11 carbon atoms, R¹ has 2 carbon atoms and R₂ and R3 each have 1 carbon atom.

The deposition aid can also be a salt such as sodium chloride, ammonium chloride or sodium sulfate or a low molecular weight organic salt, such as trisodium sulfosuccinate, sodium glycolate or triethanolamine hydrochloride, which enhances the deposition of the microemulsion through the salting out phenomena described hereinbelow and can be monomeric or polymeric.

The deposition aid can be an acidic or basic material which functions to enhance deposition by altering the pH of the formulation.

The deposition aid can also be coincidentally a material used to boost foam such as cocamide dea or a material to pearlize shampoos such as ethylene glycol monostearate or distearate. The deposition aid that is especially preferred for the compositions of this invention are nonionic lipophilic non-polar or semipolar compounds such as the mineral oils, isoparrafins, silicones, amides, amine oxides, quaternary amine functional materials and polyethylene oxides described hereinabove.

According to the present invention, the deposition aid can be a conditioner and/or softener which are defined as materials which deposit onto a surface via electrostatic or lipophilic/hydrophobic bonding. For the deposition aid component of this invention, the preferred level of the ingredient is from about 0.1 to 10 weight percent based on the total weight of the composition.

The deposition aid of the present invention can be monomeric or polymeric in form. Optionally, the deposition aid component described above can also be a part of Component (A) described hereinabove.

Component (C) in the Compositions of the present invention is water and forms the remainder of the composition. It is generally present at a level of from about 40% to about 95%, preferably from about 60% to about 90%.

The hair care compositions of this invention may form a shampoo, in which case, the compositions contain a cleansing agent in addition to the ingredients mentioned hereinabove. The concentration of cleansing agent can range from 5 to 50 parts by weight per 100 parts of total composition. Detersive surfactants selected from the group consisting of the anionic and nonionic types are well known for use in shampoo formulations. For example, the anionic and nonionic surfactants useful as cleansing agents in shampoos are further described in U.S. Patent No. 4,559,227, issued December 17, 1985. The detersive surfactants incorporated hereinabove also function as a foaming agent in the shampoo compositions of the present invention. The identity of the detersive surfactant in the shampoo compositions of this invention is not critical as long as the surfactant system in the shampoo is capable of cleaning the hair and providing an acceptable level of foam on the hair. The surfactant system may comprise one or more soluble detergents, i.e. an anionic or nonionic surfactant which produces an acceptable level of foam and cleaning for the hair.

Typical cleansing surfactants include anionic surfactants such as the sodium, ammonium or triethanolamine salts of lauryl sulfate and lauryl ether sulfate and nonionic surfactants such as fatty acid alkanolamides like lauric acid diethanolamide. Especially preferred anionic surfactants are the sodium, magnesium, ammonium and the mono-, di- and triethanolamine salts of alkyl and aralkyl sulfates, as well as these salts of alkaryl sulfonates since they provide richer, denser foams than other types of cleansing surfactants at comparable concentrations. The alkyl groups of the detergents generally have a total of from about 12 to 21 carbon atoms, may be unsaturated and are preferably fatty alkyl groups. The sulfates may be sulfate ethers containing one to ten ethylene oxide or propylene oxide units per molecule. Preferably, the sulfate ethers contain 2 to 3 ethylene oxide units. Sodium lauryl ether sulfate is also very suitable for use in the compositions of this invention.

Especially preferred nonionic surfactants include lauric acid diethanolamide, N-lauryl dimethylamine oxide, coconut acid diethanolamide, myristic acid diethanolamide and oleic acid diethanolamide. The cleaning agent of the present invention can be identical to the deposition aid in certain permutations of this invention. The cleaning agent can be a nonionic or anionic surfactant as described hereinabove. The deposition aid of the present invention can be a nonionic, cationic or anionic compound as described hereinabove. Therefore, this invention does contemplate that if desired, the deposition aid and cleaning agent could both be an identical nonionic compound or the deposition aid and cleaning agent could be an identical anionic compound. The reason this is true is that some of the cleaning agents that are contemplated by the present invention have the ability to act as deposition aids as well as detersive surfactants.

The next ingredient in the hair care formulations of the present invention is an optional water-thickening agent selected from the group consisting of electrolytes, polyelectrolytes and water soluble polymers. A thickener is preferred in the hair care formulations of the present invention to facilitate the hand application of the hair care formula to the hair. Thickeners are preferably in sufficient quantities to provide a convenient viscosity. For example, viscosities within the range of 1,000 to 30,000 cps or more preferably in the range of 3,000 to 7,000 cps as measured at 25°C. are usually suitable. Suitable thickeners include, among others, sodium alginate, gum arabic, polyoxyethylene, guar gum, hydroxypropyl guar gum, cellulose derivatives such as methylcellulose, methylhydroxypropylcellulose, hydroxypropylcellulose, polypropylhydroxyethylcellulose, starch and starch derivatives such as hydroxyethylamylose and starch amylose, locust bean gum, electrolytes such as sodium chloride, ammonium chloride, methocel and saccharides such as fructose and glucose and derivatives of saccharides such as PEG-120 methyl glucose diolate. Preferred thickeners include the cellulose derivatives, saccharide derivatives and electrolytes. It is especially preferred to use a combination of cellulose and/or starch derivatives and any electrolyte.

In accordance with the present invention, "salting out" is used to describe a phenomena whereby a solubilized or dispersed species becomes insolubilized or non-dispersed upon the addition of ionic strength or "salt". The ionic strength may be due to the addition of electrolyte (e.g. NaCl, NH₄Cl, etc.) or poly-electrolyte (e.g. ionic polymers) or both.

Other optional components may be added to the hair care compositions of this invention such as perfumes, pH control ingredients, antimicrobials, antioxidants, ultraviolet light absorbers and medicaments. The perfumes which can be used in the hair care compositions are the cosmetically acceptable perfumes and they may be present in amounts which vary from 0.1 to 0.5 percent by weight.

Although not required, it is preferred to employ an acid to adjust the pH within the range of 4 to 9 or more preferably within the range of 4.5 to 7 in the hair care compositions of this invention. Any water soluble acid such as a carboxylic acid or a mineral acid is suitable. For example, suitable acids include mineral acids such as hydrochloric, sulfuric and phosphoric; monocarboxylic acid such as acetic acid, lactic acid; and polycarboxylic acids such as succinic acid, adipic acid and citric acid.

The hair care compositions of the present invention may be in the form of a gel, paste or a freely pourable liquid. The hair care compositions can be used on the hair of humans or animals to cleanse and improve the appearance of their or coats, respectively. The shampoo compositions of the present invention are expected to be used by the usual method of adding the shampoo to the hair, massaging the shampoo into the hair and removing the shampoo from the hair by rinsing with water.

The hair care compositions of the present invention have superior stabilities during storage and shipping. During the shampooing operation, the shampoos provide a rich and billowy lather. After the shampoo is rinsed from the hair, the hair is left with a clean feeling but at the same time it is conditioned so that it is more easily combed than the hair washed shampoos without the amino-functional microemulsion component.

Another advantage of the hair care compositions of the present invention is that the amino-functional microemulsion conditioning component is quite effectively delivered, i.e., deposited on the hair from the hair care composition. In view of the fact that the cationic and nonionic lipophilic compounds of the prior art do not generally deposit well from hair care compositions containing anionic detersive surfactants, it is surprising that the amine functional microemulsion and the cationic or nonionic lipophilic compounds (as part of the microemulsion or separately) is so effectively deposited from the hair care compositions of the present invention which can contain anionic detersive surfactants and remain clear and stable. Since anionic detergents are preferred for reasons already discussed, the hair care compositions of the present invention containing anionic detergents provide a unique and advantageous combination of superior foaming and conditioning in one product. The hair care formulations herein can contain a variety of nonessential optional components suitable for rendering such formulations more acceptable. The hair care formulation may be prepared by simply mixing all ingredients together and stirring them thoroughly. This method is advantageous over the art cited above which requires the use of sophisticated suspension technology or laborious premixing or pretreatment in the case of clear systems. Heat may be applied to improve the dispersion of the ingredients.

This invention accordingly also provides a method of making an optically clear hair care composition including at least one hair care component blended with a composition comprising (A) a polyorganosiloxane microemulsion, (B) a deposition aid and (C) water, wherein the polyorganosiloxane microemulsion is as defined above. The invention accordingly also provides a method of treating hair by applying to the hair a formulation including at least one shampoo component blended with a composition comprising, (A) a polydiorganosiloxane microemulsion, (B) a deposition aid and (C) water, wherein the polyorganosiloxane microemulsion is as defined above. The invention accordingly also provides a method of shampooing hair by (I) wetting the hair, (II) applying a shampoo composition of this invention to the hair, (III) working the composition into the hair and (IV) rinsing the composition from the hair.

The turbidity of the liquid samples in the examples delineated below was tested by light scattering according to Corporate Test Method (CTM) 0851 which is described hereinbelow and a copy of which is included with this specification. Turbidity or haze of liquids caused by the presence of suspended particulate matter is measured by light scattering. Light is passed through a flat bottom cell containing the liquid. As the light beam strikes particles in the liquid, some of the light is scattered at right angles to the incident beam and is received by a photomultiplier tube. The photomultiplier tube converts the light energy into an electrical signal which is measured on a meter. The unit of measure is the Nephelometric Turbidity Unit, NTU and is based on formazin suspensions as standards. The range of turbidities detectable is 0.04 to 1,000 NTU's. Extremely turbid or highly colored solutions may produce low readings.

The viscosity of the liquids in the examples below were in a glass capillary viscometer according to Corporate Test Method (CTM) 0004. The kinematic viscosity of liquids is determined by measuring the time required for a fixed volume of samples to pass through a calibrated glass capillary using "gravity-flow". The method is based on ASTM D-445, IP 71 and the results are reported in Stokes. A copy of the test method is attached to this specification for further reference.

Polyquaternium-11 is polyvinylpyrrolidone/polydimethyl aminoethylmethacrylate dimethylsulfate quaternary ammonium salt. Cocamide DEA is coconut acid diethanolamide. Nonoxynol-4 is polyoxyethylene (4) nonyl phenyl ether.

It will be apparent from the foregoing that many other variations and modifications may be made in the compounds, compositions and methods described herein without departing substantially from the essential features and concepts of the present invention. Accordingly, it should be clearly understood that the forms of the invention described herein are exemplary only.

Silicone Microemulsion A is a translucent, cationic microemulsion having an average droplet size of 30 nm diameter. The emulsion was prepared by emulsion polymerization (the method of U.S. application for patent, Serial No. 532,471, filing date June 1, 1990 and assigned to the same assignee as this present application, as described hereinabove) of dimethylsiloxane cyclics and contained about 30% hydroxy terminated amino functional polydimethylsiloxane and about 3% of unpolymerized dimethylsiloxane cyclics. The emulsion was stabilized by 3% tallowtrimethylammoniumchloride, a cationic surfactant and an ethoxylated lauryl alcohol, a nonionic surfactant. The silicone microemulsion also contained a preservative (Chloromethyl Isothiazolin-3-One).

Silicone Microemulsion B is a translucent, cationic microemulsion having an average droplet size of 30nm diameter. The emulsion was prepared by emulsion polymerization (the method of U.S. application for patent, Serial No. 532,471, filing date June 1, 1990 and assigned to the same assignee as this present application, as described hereinabove) dimethylsiloxane cyclics and contained about 30 % hydroxy terminated diamino functional polydimethylsiloxane and about 3% of unpolymerized dimethylsiloxane cyclics. The emulsion was stabilized by 3% tallowtrimethylammoniumchloride, a cationic surfactant and an ethoxylated lauryl alcohol, a nonionic surfactant. The silicone microemulsion also contained a preservative (Chloromethyl Isothiazolin-3-One).

Silicone Microemulsion C is a transparent, nonionic microemulsion having an average droplet size of 30nm diameter. The emulsion was prepared by the mechanical method of U.S. Patent No. 4,620,878. The composition contains about 15% of a 400DP, 2 mol %, amino functional polydimethylsiloxane, about 0.3% polyethoxylated decyl alcohol, about 3.2% of a tridecethcarboxylic acid, about 0.3% triethanolamine, about 7% octylphenoxy polyethoxy (40) ethanol (70 percent in water) and about 74% water.

Silicone Microemulsion D is a transparent, nonionic microemulsion having an average droplet size of 30nm diameter. The emulsion was prepared by the mechanical method of U.S. Patent No. 4,620,878. The composition contains about 15% of a 400DP, 2 mol%, amino functional polydimethylsiloxane, about 2.5% trimethyl nonyl polyethylene glycol ether about 3.9% of a carboxylic acid, about 0.3% triethanolamine, about octylphenoxy polyethoxy (40) ethanol (70 percent in water) and about 68% water.

Silicone Microemulsion E is a transparent, anionic microemulsion having an average droplet size of 25nm diameter. The emulsion was prepared by emulsion polymerization of dimethylsiloxane cyclics and contained about 18% hydroxy terminated amino functional polydimethylsiloxane, about 2% of unpolymerized dimethysiloxane cyclics, 7.7% tallowtrimethylammoniumchloride, a cationic surfactant and 5.6% of polyoxyethylene (40) octylphenyl ether, a nonionic surfactant. The silicone microemulsion also contained a preservative (Chloromethyl Isothiazolin-3-One).

Silicone Microemulsion F is a cloudy yellowish cationic microemulsion having an average droplet size of 65nm diameter. The emulsion was prepared by emulsion polymerization (the method of U.S. application for patent, Serial No. 532,471, filing date June 1, 1990 and assigned to the same assignee as this present application, as described hereinabove) of dimethylsiloxane cyclics and contained about 30 % hydroxy terminated amino functional polydimethylsiloxane, about 2% unpolymerized dimethylsiloxane cyclics, 4.7 % of a secondary alcohol ethoxylate, a nonionic surfactant. The composition contained about 55% water. The silicone microemulsion also contained a preservative (Chloromethyl Isothiazolin-3-One).

Silicone Microemulsion G is a clear to slightly yellowish cationic microemulsion having an average droplet size of 30nm diameter. The emulsion was prepared by the emulsion polymerization of dimethylsiloxane cyclics and contained about 30% hydroxy terminated amino functional polydimethylsiloxane and about % of unpolymerized dimethylsiloxane cyclics. The emulsion was stabilized by 5.8% tallowtrimethylammoniumchloride, a cationic surfactant and an ethoxylated nonyl alcohol, a nonionic surfactant. The silicone microemulsion also contained a preservative (Chloromethyl Isothiazolin-3-One).

Silicone Microemulsion H is a transparent, nonionic microemulsion. The emulsion was prepared mechanically by mixing about 20% of a 400 DP, 2mol % amino functional polydimethylsiloxane, about 3% of a nonionic surfactant, trimethyl nonyl polyethylene glycol ether, about 2% of a nonionic surfactant, a polyethylene glycol ether of a linear alcohol, about .4% acetic acid and about 74.6% water.

Silicone Microemulsion I is a nonionic microemulsion containing 30% of 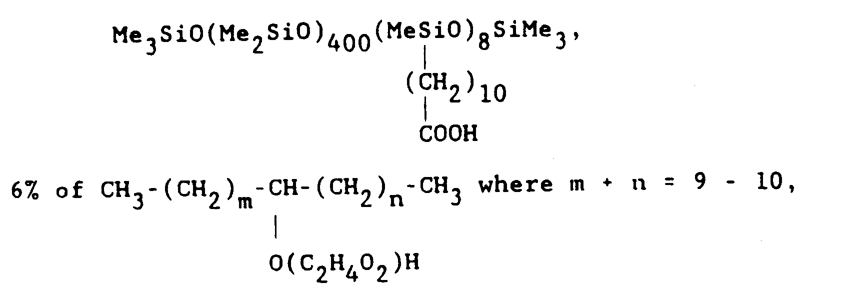
2% ethoxylated trimethylnonanol(a nonionic surfactant), 0.4% sodium carbonate, 0.1% sodium benzoate, 0.1% of a mixture of hexamethylenetetramine and 1,3-dichloropropene salt (a preservative) and 61.4% water.

### EXAMPLE I

The following Dip Bath compositions were then prepared from the above described microemulsions in combination with a deposition aid and these compositions are described in Table I below.

**TABLE I**

| DIP BATH COMPOSITIONS | | |
|---|---|---|
| DIP BATH COMPOSITION | MICROEMULSION | DEPOSITION AID |
| J | - | water |
| K | - | 0.4 % dicocodimethylammonium chloride |
| L | 2% E | - |
| M | 2% E | 0.4 % dicocodimethylammonium chloride |
| N | 2% F | 0.4 % dicocodimethylammonium chloride |
| O | 2% C | - |
| P | 2% B | 0.4 % dicocodimethylammonium chloride |
| Q | 2% G | 0.4 % dicocodimethylammonium chloride |
| R | 2% A | 0.4 % dicocodimethylammonium chloride |
| S | 2% A | 0.4 % dicocodimethylammonium chloride |
| T | 2% C | 0.4 % dicocodimethylammonium chloride |
| U | 2% B | 0.4 % dicocodimethylammonium chloride |
| V | 2% C | 0.4% Cocamide DEA |
| W | 2% C | 0.4% polyquaternium-11 |
| X | 2% C | 0.4% Nonoxynol-4 |
| Y | 2% C | 0.4% Sodium laureth (3) sulfate |

The above Dip Bath Compositions (J-Y) were then evaluated for hair conditioning by the method described hereinbelow.

Two gram hair tresses, on tabs, were prepared, shampooed with a blank shampoo, dried and then subjected to a 30 minute presoak in distilled water. Each tress was removed, the excess water was removed and the turn was detangled and then re-tangled by dipping it into distilled water three times over 15 seconds. Excess water was again removed. Each tress was dipped into a 200g base, with the test material diluted to 2%, for 30 seconds. Each tress was removed, excess water removed and rinsed for 30 seconds in 40 C tap water. The excess water removed, detangling and re-tangling steps were repeated. Each tress was rated for wet combing (wc), wet feel (wf), dry combing (dc), dry feel (df) and appearance after zero, five and ten shampoo latherings. In the rating system, 1=best and 5=worst. The results of these tests are detailed below in Table II.

**TABLE II**

| SUBSTANTIVE CONDITIONING | | | | | | | | | | | | |
|---|---|---|---|---|---|---|---|---|---|---|---|---|
| Treatment | Initial | | | | After 5 Shampoos | | | | After 10 shampoos | | | |
| | wc | wf | dc | df | wc | wf | dc | df | wc | wf | dc | df |
| J | 3 | 2.5 | 3 | 3 | 4 | 3 | 3.5 | 3 | 5 | 3 | 3.5 | 3 |
| K | 1.5 | 2 | 2 | 2.5 | 4 | 3 | 1.5 | 3 | 5 | 3 | 3 | 3 |
| L | 3 | 2.5 | 2 | 2 | 3 | 3 | 3 | 3 | 4.5 | 3 | 3 | 3 |
| M | 1 | 2 | 1 | 1.5 | 2 | 3 | 2 | 2.5 | 4.5 | 3 | 3 | 3 |
| N | 1 | 2 | 1 | 2 | 1 | 2 | 2.5 | 2.5 | 5 | 3 | 2.5 | 3 |
| O | 3.5 | 3 | 2.5 | 2.5 | - | - | - | - | - | - | - | - |
| P | 1.5 | 3 | 1.5 | 2.5 | - | - | - | - | - | - | - | - |
| Q | 1.5 | 2 | 1.5 | 2 | 2 | 2 | 1.5 | 2 | 2.5 | 3 | 2.5 | 3 |
| R | 1.25 | 2 | 1 | 2 | - | - | - | - | - | - | - | - |
| S | 1 | 2 | 1.5 | 1.5 | 1.25 | 2 | 1.5 | 2 | 1.5 | 2 | 1.75 | 2.5 |
| T | 1.5 | 3 | 1 | 2.5 | 1 | 2 | 1.25 | 2 | 1.5 | 2 | 1.5 | 2.5 |
| U | 1.5 | 2 | 1.5 | 2.5 | - | - | - | - | - | - | - | - |
| V | 3 | 3 | 2 | 2 | 3 | 2 | 2 | 2 | 2 | 2 | 3 | 2 |
| W | 1 | 2 | 2 | 2 | 3 | 2 | 2.5 | 2 | 3 | 1 | 3 | 2 |
| X | 2.5 | 3 | 1 | 2 | 2.5 | 2 | 2 | 2 | 3.5 | 2 | 2.5 | 2.5 |
| Y | 2.5 | 2 | 1 | 2 | 3 | 2 | 2.5 | 3 | 4 | 2 | 3 | 2.5 |

This example illustrates the benefit of substantive conditioning when an amino functional microemulsion is used in combination with a deposition aid. This example also illustrates the following order of preference of microemulsion/deposition aid to obtain the most effective substantive conditioning going from most effective to least effective: aminofunctional microemulsion/quaternary deposition aid > aminofunctional microemulsion/nonionic deposition aid > aminofunctional microemulsion/polyquaternary deposition aid > aminofunctional microemulsion/nonionic polyethylene oxide deposition aid > aminofunctional microemulsion/anionic deposition aid. In all cases, the aminofunctional microemulsion is preferred over the polydimethylsiloxane microemulsion which demonstrated no substantive conditioning even when combined with a deposition aid. This example also shows the improvements gained when using aminofunctional microemulsions with a deposition aid over using either ingredient alone.

### EXAMPLE II

The following samples were prepared as described hereinbelow.

A shampoo composition (Sample I) was prepared by mixing 670g of water, 10g hydroxypropylmethylcellulose, 350g of 30% ammonium lauryl sulfate and 30g of cocamide DEA (coconut acid diethanolamide).

A second shampoo composition (Sample 2) was prepared by mixing 670g of water, 350g of 30% ammonium lauryl sulfate and 30g of cocamide DEA.

A pre-gel composition (Sample 3) was prepared by mixing 995g of water and 5g of carbomer 934 (need CTFA name).

A conditioner composition (Sample 4) was prepared by mixing 985g of water and 15g hydroxyethylcellulose.

A shampoo composition (Sample 5) was prepared as in sample 2, except that the pH of that composition was adjusted to 6 with aqueous citric acid, then ammonium chloride was added to the mixture until a viscosity of 7000 cs was reached.

A shampoo composition (Sample 6) was prepared by mixing 50g sodium lauryl ether (2) sulfate, 2g lauramide DEA (lauric acid diethanolamide) and 48g water, then adjusting the pH of the mixture to 6 with citric acid and then adding sodium chloride to the mixture until a viscosity of 7000 cs is reached.

A shampoo composition (Sample 7) was prepared by mixing 18g of the shampoo of sample 2, 1.1g of Microemulsion H, adjusting the pH of the mixture to 6 with Citric Acid, then adding 0.10g of 0.5% Ammonium Chloride.

A shampoo composition (Sample 8) was prepared by mixing 18g of the shampoo of sample 2, 1.1g of Microemulsion E, adjusting the pH of the mixture to 6 with Citric Acid, then adding 0.10g of 0.5% Ammonium Chloride.

A shampoo composition (Sample 9) was prepared by mixing 18g of the shampoo of sample 2, 0.7g of Microemulsion H, adjusting the pH of the mixture to 6 with Citric Acid, then adding 0.10g of Ammonium Chloride.

A shampoo composition (Sample 10) was prepared by mixing 18g of the shampoo of sample 2, 0.7g of Microemulsion E, adjusting the pH of the mixture to 6 with Citric Acid, then adding 0.10g of Ammonium Chloride.

A shampoo composition (Sample 11) was prepared by mixing 18g of the shampoo of sample 2, 0.35g of Microemulsion H, adjusting the pH of the mixture to 6 with Citric Acid, then adding 0.10g of Ammonium Chloride.

A shampoo composition (Sample 12) was prepared by mixing 18g of the shampoo of sample 2, 0.35g of Microemulsion E, adjusting the pH of the mixture to 6 with Citric Acid, then adding 0.10g of Ammonium Chloride.

A shampoo composition (Sample 13) was prepared by mixing 15g of the shampoo of sample 2, 0.9g of Microemulsion C, adjusting the pH of the mixture to 5 with Citric Acid, then adding 0.10g of 0.67% Ammonium Chloride.

A shampoo composition (Sample 14) was prepared by mixing 15g of the shampoo of sample 2, 0.9g of Microemulsion D, adjusting the pH of the mixture to 5 with Citric Acid, then adding 0.10g of 0.67% Ammonium Chloride.

A shampoo composition (Sample 15) was prepared by mixing 60g of the shampoo of sample 2, 3.6g of Microemulsion F, adjusting the pH of the mixture to 5.6 with Citric Acid, then adding 0.28g of Ammonium Chloride.

A shampoo composition (Sample 16) was prepared by mixing 60g of the shampoo of sample 2, 3.6g of Microemulsion A, adjusting the pH of the mixture to 5.8 with Citric Acid, then adding 0.24g of Ammonium Chloride.

A shampoo composition (Sample 17) was prepared by mixing 60g of the shampoo of sample 2, 3.6g of Microemulsion G, adjusting the pH of the mixture to 5.8 with Citric Acid, then adding 0.24g of Ammonium Chloride.

A shampoo composition (Sample 18) was prepared by mixing 30g of the shampoo of sample 1, 1.8g of Microemulsion E, adjusting the pH of the mixture to 5 with Citric Acid, then adding 0.21g of Ammonium Chloride.

A shampoo composition (Sample 19) was prepared by mixing 30g of the shampoo of sample 1, 1.8g of Microemulsion F, adjusting the pH of the mixture to 5 with Citric Acid, then adding 0.16g of Ammonium Chloride.

A shampoo composition (Sample 20) was prepared by mixing 30g of the shampoo of sample 1, 1.8g of Microemulsion A, adjusting the pH of the mixture to 5 with Citric Acid, then adding 0.16g of Ammonium Chloride.

A shampoo composition (Sample 21) was prepared by mixing 30g of the shampoo of sample 1, 1.8g of Microemulsion G, adjusting the pH of the mixture to 5 with Citric Acid, then adding 0.16g of Ammonium Chloride.

A shampoo composition (Sample 22) was prepared by mixing 30g of the shampoo of sample 1, 0.6g of Microemulsion C, adjusting the pH of the mixture to 5 with Citric Acid, then adding 0.16g of Ammonium Chloride.

A shampoo composition (Sample 23) was prepared by mixing 30g of the shampoo of sample 1, 1.2g of Microemulsion C, adjusting the pH of the mixture to 5 with Citric Acid, then adding 0.21g of Ammonium Chloride.

A shampoo composition (Sample 24) was prepared by mixing 30g of the shampoo of sample 1, 1.8g of Microemulsion C, adjusting the pH of the mixture to 5 with Citric Acid, then adding 0.16g of Ammonium Chloride.

A shampoo composition (Sample 25) was prepared by mixing 5g of Microemulsion C with 5g of 30% of sodium lauryl ether sulfate. A bluish hazy solution was formed. This sample was prepared according to Example 24 of U.S. Patent No. 4,620,878.

The shampoo compositions described above were then tested utilizing the following procedure. Hair tresses were shampooed with a Shampoo Blank and then dried. Next, 0.5g of the test shampoo was applied to the hair after it was rewetted. The shampoo was lathered on the hair with rubbing for 30 seconds and then rinsed off in running tap water for 30 seconds. Each tress was detangled by hand combing (once through with a wide part of the comb). The hair tresses were then hung up to dry. The shampoos were then evaluated after 24 hours and tested for clarity (through visual observation), wet combing(wc), wet feel(wf), dry combing(dc), dry feel(df). The results of the above described test are detailed in Table IV as shown below.

**TABLE IV**

| SHAMPOO / CONDITIONING | | | | | |
|---|---|---|---|---|---|
| Shampoo Composition (SAMPLE) | Clarity | Initial wc | wf | dc | df |
| 5 | clear | 3.5 | 3 | 3 | 3 |
| 6 | clear | 4 | 2.5 | 3 | 3 |
| 7 | clear | 1.5 | 2 | 2 | 2 |
| 8 | slight haze | 2.5 | 2.5 | 2 | 2 |
| 9 | clear | 2 | 2 | 2 | 2 |
| 10 | slight haze | 3 | 3 | 2 | 2 |
| 11 | clear | 3 | 3 | 2.5 | 3 |
| 12 | slight haze | 3.5 | 3 | 2 | 3 |
| 13 | clear | 1.75 | 2 | 1.5 | 2 |
| 14 | clear | 3 | 2 | 1.5 | 2 |
| 15 | opaque | - | - | - | - |
| 16 | clear-thin | - | - | - | - |
| 17 | opaque | - | - | - | - |
| 18 | slight haze | - | - | - | - |
| 19 | opaque | - | - | - | - |
| 20 | clear | 4 | 2 | 2 | 2 |
| 21 | opaque | - | - | - | - |
| 22 | clear | 4 | 3 | 1.5 | 2 |
| 23 | clear | 2 | 2 | 1.5 | 1.5 |
| 24 | clear | 1.5 | 2 | 2 | 1 |
| 25 | hazy | 4 | 3 | 3 | 3 |

This example illustrates the preference of aminofunctional microemulsions over polydimethylsiloxane microemulsions for shampoo clarity. It also shows the preference for a cationic equivalence of less than 0.15 for the aminofunctional microemulsions. It also illustrates the fact that the aminofunctional microemulsion is preferred to be used at less than 50%, but greater than or equal to 4% in the shampoo for clarity, good aesthetics and combing. It shows that when the aminofunctional microemulsions are used at a concentration of between 4 and 50 percent with a nonionic or cationic deposition aid, aminofunctional microemulsions unexpectedly provide superior shampoo clarity, combing and aesthetic properties when compared to those in the art.

### EXAMPLE III

This example illustrates the clarity of the compositions of this invention. Samples 15-18 and 22-24 of Example II were tested for their turbidity using a Model No. 2100A laboratory turbidimeter from Hach Chemical Company, Ames, Iowa; cells from Hach Chemical Company, catalog No. 4229.32 and Turbidity Standardization Kit, catalog No. 12684-00 from Hach Chemical Company, Ames, Iowa. The results of these tests are described in Table V below.

**TABLE V**

| TURBIDITY OF SAMPLES | | |
|---|---|---|
| SAMPLE | CLARITY | NTU'S(NEPHELOMETRIC TURBIDITY UNITS) |
| 15 | opaque | >2,000 |
| 16 | clear, thin | 4.63 |
| 17 | opaque | 53.1 |
| 18 | slighthaze | 32.6 |
| 22 | clear | 4.73 |
| 23 | clear | 7.99 |
| 24 | clear | 7.24 |

This example further illustrates that the use of a nonionic or a cationic deposition aid in combination with aminofunctional microemulsions having low cationic equivalence produces a turbidity of less than 10 NTU's which provides a clear shampoo. It is further shown that the polydimethylsiloxane microemulsions produce nonclear shampoos.

### COMPARATIVE EXAMPLE I

This example further illustrates the aesthetic value and clarity of the present invention over the compositions of the art. Samples 23, 24 and 25 were further tested for clarity (through visual observation), wet combing(wc), wet feel(wf), dry combing(dc), dry feel(df), viscosity (according to CTM 0004 described hereinabove and a copy of which is attached to this specification) in centistokes and aesthetics. The results of these tests are shown in Table VI below.

**TABLE VI**

| SHAMPOO CHARACTERISTICS | | | | | | | |
|---|---|---|---|---|---|---|---|
| Sample | clarity | wc | wf | dc | df | viscosity | Aesthetics |
| 25 | very hazy | 4 | 3 | 3 | 3 | 4.79 cs | hair is heavy,combing drag, high static |
| 23 | clear | 2 | 2 | 1.5 | 1.5 | 922.9 | hair is light, easy combing, low static |
| 24 | clear | 1.5 | 2 | 2 | 1 | 155.8 | Hair is light, easily combed, low static |

The results of Comparative Example I show that the shampoo of Sam.(Sample) 25 was hazy, too thin which resulted in the shampoo running off the hair and hands and that it was too heavily released onto the hair resulting in heavily coated, limp and static filled hair after its application and use. In contrast, the application and use of the shampoos of Samples 23 and 24 resulted in an acceptible viscosity formulation such that a light coating was released onto the hair, the formulation clear and the hair was left well conditioned after its use. Therefore, Samples 23 and 24 represent operative shampoos while Sample 25 does not.

### EXAMPLE IV

The following samples were prepared by mixing the ingredients described in Table VII below. The samples were then looked at to see the amount of preparation time it took to form a hair care composition.

**TABLE VII**

| SALT CURVE DETERMINATION | | | |
|---|---|---|---|
| Composition | SAMPLE 26 | SAMPLE 27 | SAMPLE 28 |
| water | 118.3 | 118.3 | 125.5 |
| 30% ammonium lauryl sulfate | 66.7 | 66.7 | 66.7 |
| Cocamide DEA | 9.0 | 9.0 | 9.0 |
| Microemulsion E | 9.0 | - | - |
| Microemulslon C | - | 12.0 | - |
| Trimethylsilylamodimethicone | - | - | 1.8 |
| Method of Addition | Add-in | Add-in | premix |
| Time required to prepare | 15 min. | 15 min. | 24 hours |
| Citric Acid(30%) | pH 5.7 | pH 5.7 | pH 5.7 |

| Ammonium Chloride | | | |
|---|---|---|---|
| A | 0 | 0 | 0 |
| B | 0.15g | 0.15g | 0.15g |
| C | 0.45g | 0.45g | 0.45g |
| D | 0.84g | 0.78g | 0.80g |
| E | 1.10g | 1.11g | 1.05g |
| F | 1.35g | 1.37g | 1.37g |

This example illustrates the ability of the aminofunctional microemulsions to be used by simply mixing the ingredients in contrast to some compositions of the art which require a premixing step which takes 24 hours to produce a shampoo composition.

### EXAMPLE V

Samples 26-28 of Example IV were then prepared by mixing an amount of sample and an amount of ammonium chloride as described in TABLE VIII below. These formulations were prepared to test each sample's resistance to the salting out phenomenon described hereinabove. The samples were tested for their appearance(visually) after 24 hours, their viscosity (CTM 0004 described hereinabove) and their clarity (haze value) according to CTM 0851 also described hereinabove. The results are displayed in Table VIII below.

**TABLE VIII**

| SALTING OUT DETERMINATION | | | | |
|---|---|---|---|---|
| SAMPLE | %NH4Cl | APPEARANCE (24 HOUR) | VISCOSITY(cs) (72 hour) | HAZE (NTU'S) |
| 26A | 0 | slightly hazy | 39.4 | 22.9 |
| 26B | 0.5 | slightly hazy | 920.2 | 25.8 |
| 26C | 1.5 | slightly hazy | 932 | 19.5 |
| 26D | 2.8 | slightly hazy | 65 | 11.01 |
| 26E | 3.667 | separates | 41.5 | 16.1 |
| 26F | 4.5 | separates | 16.7 | 22.3 |
| 27A | 0 | clear | 23.92 | 2.42 |
| 27B | 0.5 | clear | 108.4 | 3.36 |
| 27C | 1.5 | clear | 284 | 5.94 |
| 27D | 2.6 | clear | 128.3 | 11.90 |
| 27E | 3.7 | oiling out | 72.7 | 27.0 |
| 27F | 4.567 | opaque | 62.8 | >2000 |
| 28A | 0 | clear | 11.02 | 2.16 |
| 28B | 0.5 | clear | 29.83 | 3.41 |
| 28C | 1.5 | clear | 477 | 6.09 |
| 28D | 2.667 | clear | 210.9 | 9.85 |
| 28E | 3.5 | clear | 124.4 | 14.6 |
| 28F | 4.567 | oiling out | 91.2 | 30.4 |

This example illustrates the ability of the present invention to resist salting out to comparable degrees as compositions existing in the art. It also illustrates the preference for aminofunctional microemulsions over polydimethylsiloxane microemulsions to attain shampoo clarity.

### EXAMPLE VI

The following hair care formulations were prepared by mixing the ingredients described in Table IX below. This example compares the characteristics of a hair care composition containing aminofunctional microemulsions in contrast to compositions containing polydimethylsiloxane microemulsions and microemulsions containing carboxylic acid functionality. Samples 29-32 were tested for appearance (through visual observation), wet combing(wc), wet feel(wf), dry combing(dc), dry feel(df), viscosity (according to CTM 0004 described hereinabove and a copy of which is attached to this specification) in centistokes and clarity (according to Corporate Test Method (CTM) 0851 described hereinabove). The results are shown in Table IX below.

**TABLE IX**

| CHARACTERISTICS OF HAIR CARE COMPOSITION | | | | |
|---|---|---|---|---|
| Composition | SAMPLE 29 | SAMPLE 30 | SAMPLE 31 | SAMPLE 32 |
| SAMPLE 5 (unneutralized, no salt) | 30g | 30g | 30g | 30g |
| Microemulsion E | 1.35g | - | - | - |
| Microemulsion C | - | 1.8g | - | - |
| Microemulsion I | - | - | 1.8g | - |
| Citric Acid | pH 5.8 | pH 5.8 | pH 5.8 | pH 5.8 |
| Ammonium Chloride | .11 | .11 | .10 | .10 |

| RESULTS | | | | |
|---|---|---|---|---|
| Appearance | slight haze | clear | clear | clear |
| wet feel | 3 | 3 | 3 | 3 |
| wet combing | 3 | 1 | 3 | 4 |
| dry combing | 2 | 2 | 2.5 | 3 |
| dry feel | 2 | 2 | 2 | 3 |
| viscosity(cs) | 2906 | 412.1 | 1109 | 10,000 |
| haze(NTU's) | 22.1 | 3.48 | 4.86 | 5 |

As one can see from Table IX above, the silicone levels for samples 29-31 were the same. This example illustrates that the amino-functional microemulsion hair care compositions provide better conditioning(wet combing) than do the compositions of the art. They also provide greater clarity and a more acceptable viscosity than do the compositions of the art. The aminofunctional microemulsions provide the best overall hair care properties when compared to polydimethylsiloxane microemulsions. The carboxylic acid functional microemulsions also displayed favorable characteristics. Also, the amino-functional microemulsions of this invention unexpectedly provided a clearer composition than the blank in the above example.

### EXAMPLE VII

The following formulations were prepared by mixing the ingredients described in Table X below. This example illustrates the benefits provided by the deposition aids utilized by the present invention. These formulations were tested for appearance (through visual observation), wet combing(wc), wet feel(wf), dry combing(dc), dry feel(df), viscosity (through visual observation). The results are shown in Table X below.

**TABLE X**

| CHARACTERISTICS OF HAIR CARE COMPOSITION | | | |
|---|---|---|---|
| Composition | SAMPLE 33 | SAMPLE 34 | SAMPLE 35 |
| Water | 18.2 | 17.3 | Blank |
| Cocamide DEA | 0 | 0.9 | Sample 5 |
| Ammonium Lauryl Sulfate (30%) | 10.0 | 10.0 | - |
| Microemulsion C | 1.8 | 1.8 | - |
| Citric Acid (30%) | pH 5.2 | pH 5.2 | |
| Ammonium Chloride | 0.36 | 0.36 | - |

| RESULTS | | | |
|---|---|---|---|
| Appearance | clear homogenous | clear homogenous | clear homogenous |
| Wet Feel | 3 | 3 | 3 |
| Wet Combing | 3 | 4 | 4 |
| Dry Combing | 2 | 2 | 3 |
| Dry Feel | 2 | 2 | 3 |
| Viscosity | very thin | medium | good |

This example illustrates that Cocamide DEA can act both as a deposition aid and a viscosity builder in the present invention. This example further illustrates that the use of an aminofunctional microemulsion in combination with a nonionic deposition aid produces an optically clear hair care composition with good conditioning properties and cleaning properties in one composition.

## Claims

1. An optically clear hair care composition comprising:
(A) a polyorganosiloxane microemulsion;
(B) a deposition aid selected from the group consisting of non-polar oils selected from mineral oils, alkanes, or naturally derived oils, silicones selected from the group consisting of cyclomethicone, dimethicone, amodimethicone polymers, and dimethicone copolyols, resins, salts selected from the group consisting of a nonpolar quaternary ammonium salt having its formula selected from the group consisting of 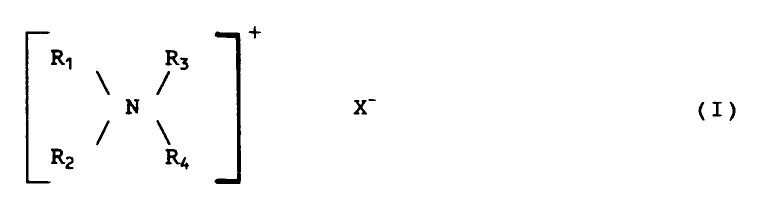 wherein R₁ is selected from the group consisting of an aliphatic group of from 12 to 22 carbon atoms, an aromatic group having from 12 to 22 carbon atoms, an aryl group having from 12 to 22 carbon atoms, and an alkylaryl group having from 12 to 22 carbon atoms, R₂ is an aliphatic group having from 12 to 22 carbon atoms, R₃ and R₄ are each alkyl groups having from 1 to 3 carbon atoms, and X is an anion selected from halogen, acetate, phosphate, nitrate and alkylsulfate radicals, and 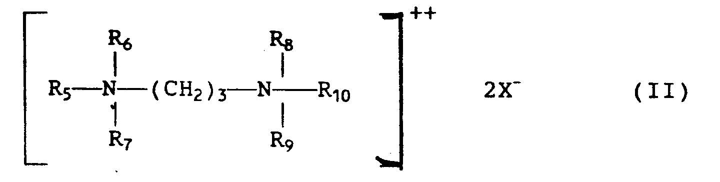 wherein R₅ is an aliphatic group having from 16 to 22 carbon atoms, R₆, R₇, R₈, R₉, and R₁₀ are selected from the group consisting of hydrogen and alkyl radicals having from 1 to 4 carbon atoms, and X is an ion selected from the group consisting of halogen, acetate, phosphate, nitrate, and alkyl sulfate radicals, amine salts, sodium chloride, ammonium chloride, sodium sulfate, and low molecular weight organic salts; and
(C) water;
wherein the polyorganosiloxane microemulsion comprises a radical selected from the group consisting of a polar radical attached to Si through Si-C or Si-O-C bonds and a silanol radical; wherein the microemulsion additionally comprises a surfactant which is insoluble in the polyorganosiloxane and water; wherein the polyorganosiloxane in the microemulsion has an average particle size of less than 0.14 µm (microns)

2. The composition according to claim 1 further comprising a cleaning agent.

3. A method of making an optically clear hair care composition comprising blending at least one hair care component with a composition of one of claims 1 or 2.

4. A method of treating hair by applying to the hair a formulation including at least one shampoo component blended with an optically clear composition of one of claims 1 or 2.

5. The method according to claim 4 further comprising additionally mixing with components (A), (B) and (C), a water-thickening agent.

6. A method of shampooing hair by
(I) wetting the hair;
(II) applying the composition as claimed in claim 2 to the hair;
(III) working the composition into the hair; and
(IV) rinsing the composition from the hair.

## Patentansprüche

1. Optisch klare Haarpflegezuammensetzung, umfassend:
(A) eine Polyorganosiloxanmikroemulsion;
(B) ein Ablagerungshilfsmittel ausgewählt aus der Gruppe bestehend aus nicht-polaren Ölen ausgewählt aus Mineralölen, Alkanen oder natürlichen Ölen, Silikonen ausgewählt aus der Gruppe bestehend aus Cyclomethicone, Dimethicone, Amodimethiconepolymeren und Dimethiconecopolyolen, Harzen, Salzen ausgewählt aus der Gruppe bestehend aus einem nicht-polaren quaternären Ammoniumsalz der Formel ausgewählt aus der Gruppe bestehend aus 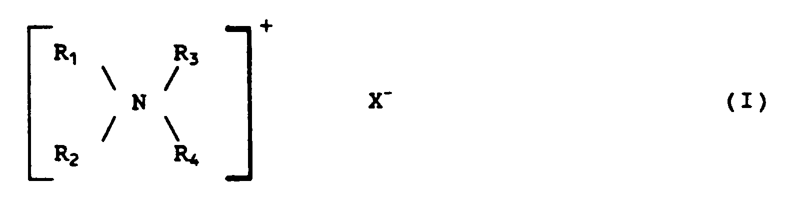 worin R₁ ausgewählt ist aus der Gruppe bestehend aus einer aliphatischen Gruppe mit 12 bis 22 Kohlenstoffatomen, einer aromatischen Gruppe mit 12 bis 22 Kohlenstoffatomen, einer Arylgruppe mit 12 bis 22 Kohlenstoffatomen und einer Alkylarylgruppe mit 12 bis 22 Kohlenstoffatomen, R₂ eine aliphatische Gruppe mit 12 bis 22 Kohlenstoffatomen ist, R₃ und R₄ jeweils Alkylgruppen mit 1 bis 3 Kohlenstoffatomen sind und X ein Anion ist ausgewählt aus Halogen-, Acetat-, Phosphat-, Nitrat- und Alkylsulfatresten und 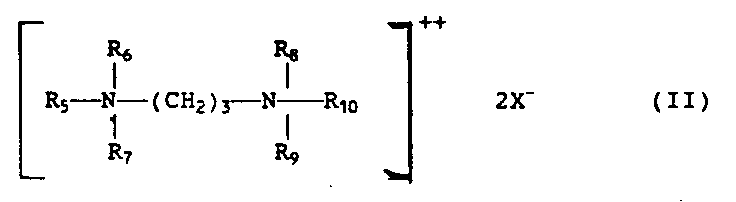 worin R₅ eine aliphatische Gruppe mit 16 bis 22 Kohlenstoffatomen ist, R₆ R₇, R₈, R₉ und R₁₀ ausgewählt sind aus der Gruppe bestehend aus Wasserstoff und Alkylresten mit 1 bis 4 Kohlenstoffatomen und X ein Ion ist ausgewählt aus der Gruppe bestehend aus Halogen-, Acetat-, Phosphat-, Nitrat- und Alkylsulfatresten, Aminsalzen, Natriumchlorid, Ammoniumchlorid, Natriumsulfat und organischen Salzen mit niedrigem Molehulargewicht; und
(C) Wasser;
wobei die Polyorganosiloxanmikroemulsion einen Rest umfaßt, ausgewählt aus der Gruppe bestehend aus einem polaren Rest, der an Si durch Si-C- oder Si-O-C-Bindungen gebunden ist, und einem Silanolrest, wobei die Mikroemulsion zusätzlich ein Tensid umfaßt, das in dem Polyorganosiloxan und Wasser unlöslich ist; wobei das Polyorganosiloxan in der Mikroemulsion eine durchschnittliche Teilchengröße von weniger als 0,14 µm (microns) hat.

2. Zusammensetzung nach Anspruch 1, weiter umfassend ein Reinigungsmittel.

3. Verfahren zur Herstellung einer optisch klaren Haarpflegezusammensetzung, umfassend, daß man mindestens eine Haarpflegekomponente mit einer Zusammensetzung nach einem der Ansprüche 1 oder 2 vermischt.

4. Verfahren zur Behandlung von Haar, indem auf das Haar ein Präparat aufgetragen wird, das mindestens eine Shampookomponente vermischt mit einer optisch klaren Zusammensetzung nach einem der Ansprüche 1 oder 2 enthält.

5. Verfahren nach Anspruch 4, weiter umfassend, daß man zusätzlich mit den Komponenten (A), (B) und (C) ein Wasserverdickungsmittel vermischt.

6. Verfahren zum Shampoonieren von Haar, indem man
(I) das Haar befeuchtet;
(II) die Zusammensetzung nach Anspruch 2 auf das Haar aufträgt;
(III) die Zusammensetzung in das Haar einarbeitet und
(IV) die Zusammensetzung aus dem Haar ausspült.

## Revendications

1. Une composition optiquement limpide pour soins capillaires, comprenant :
(A) une microémulsion de polyorganosiloxane ;
(B) un adjuvant de dépôt choisi dans la classe formée des huiles non polaires choisies parmi les huiles minérales, les alcanes ou les huiles d'origine naturelle, des silicones choisies dans la classe formée par les polymères de cyclométhicone, de diméthicone, d'amodiméthicone, et les diméthicone-copolyols, des résines, des sels choisis dans la classe formée par un sel d'ammonium quaternaire non polaire dont la formule est choisie dans la classe formée par 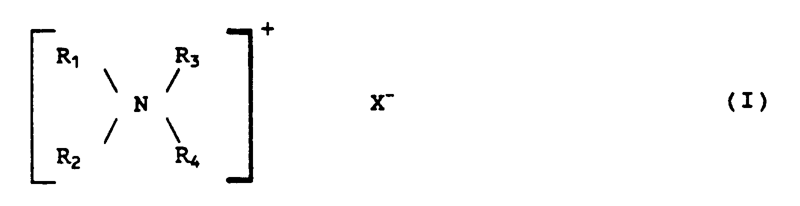 où R₁ est choisi dans la classe formée par un groupe aliphatique ayant 12 à 22 atomes de carbone, un groupe aromatique ayant 12 à 22 atomes de carbone, un groupe aryle ayant 12 à 22 atomes de carbone et un groupe alkylaryle ayant 12 à 22 atomes de carbone, R₂ est un groupe aliphatique ayant 12 à 22 atomes de carbone, chacun de R₃ et R₄ est un groupe alkyle ayant 1 à 3 atomes de carbone, et X est un anion choisi parmi les radicaux halogéno, acétate, phosphate, nitrate et alkylsulfate, et 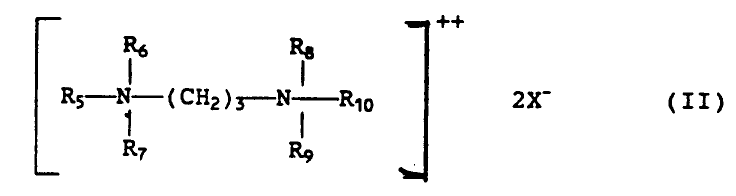 où R₅ est un groupe aliphatique ayant 16 à 22 atomes de carbone, R₆, R₇, R₈, R₉ et R₁₀ sont choisis dans la classe formée par l'hydrogène et les radicaux alkyles ayant 1 à 4 atomes de carbone, et X est un ion choisi dans la classe formée par les radicaux halogéno, acétate, phosphate, nitrate et alkylsulfate, des sels d'amines, le chlorure de sodium, le chlorure d'ammonium, le sulfate de sodium et des sels organiques de bas poids moléculaire ; et
(C) de l'eau ;
dans laquelle la microémulsion de polyorganosiloxane comprend un radical choisi dans la classe formée par un radical polaire attaché à Si par des liaisons Si-C ou Si-O-C et un radical silanol ; dans laquelle la microémulsion comprend de plus un agent tensio-actif qui est insoluble dans le polyorganosiloxane et l'eau ; dans laquelle le polyorganosiloxane contenu dans la microémulsion a une taille moyenne de gouttelettes inférieure à 0,14 µm.

2. La composition de la revendication 1, comprenant de plus un agent nettoyant.

3. Un procédé de fabrication d'une composition optiquement limpide pour soins capillaires, comprenant l'opération de mélange d'au moins un ingrédient pour soins capillaires avec une composition de l'une des revendications 1 ou 2.

4. Un procédé pour le traitement de poils ou cheveux par application aux poils ou cheveux d'une formulation comprenant au moins un ingrédient de shampooing mélangé avec une composition optiquement limpide de l'une des revendications 1 ou 2.

5. Le procédé selon la revendication 4, comprenant de plus l'opération supplémentaire de mélange d'un agent épaississant pour milieu aqueux avec les ingrédients (A), (B) et (C).

6. Un procédé pour le lavage de poils ou cheveux avec un shampooing, consistant à
(I) mouiller les poils ou cheveux ;
(II) appliquer aux poils ou cheveux la composition telle que revendiquée dans la revendication 2 ;
(III) faire pénétrer la composition dans les poils ou cheveux ; et
(IV) rincer les poils ou cheveux pour les débarrasser de la composition.
